# EUROPEAN PATENT SPECIFICATION

(11) **EP 3 668 560 B1**
(45) Date of publication and mention of the grant of the patent: **10.01.2024**
(21) Application number: 18755645.1
(22) Date of filing: 02.08.2018
(51) Int. Cl.: A61M 60/178, A61M 60/242, A61M 60/422, A61M 60/515, A61M 60/816, A61M 60/81, A61M 60/857

(54) **THROMBUS DETECTION AND REMOVAL USING A FLEXIBLE ELECTRONIC SENSOR AND EMITTER**
THROMBUSDETEKTION UND -ENTFERNUNG UNTER VERWENDUNG EINES FLEXIBLEN ELEKTRONISCHEN SENSORS UND EINES EMITTERS
DÉTECTION ET ÉLIMINATION DE THROMBUS À L'AIDE D'UN CAPTEUR ÉLECTRONIQUE FLEXIBLE ET D'UN ÉMETTEUR

(30) Priority: 18.08.2017 US 201762547354 P
(43) Date of publication of application: 24.06.2020
(73) Proprietor: Heartware, Inc., Miami Lakes, FL 33014 (US)
(72) Inventor: CASAS, Fernando, Miami Lakes, Florida 33014 (US)
(74) Representative: Zimmermann & Partner Patentanwälte mbB
(86) International application number: PCT/US2018/044907
(87) International publication number: WO 2019/036198

(56) References cited:
- DE-A1-102015 004 177
- US-A1- 2011 224 655
- US-A1- 2013 190 621
- US-A1- 2014 073 837
- US-A1- 2015 335 804

## Description

### TECHNICAL FIELD

This disclosure relates to a system for thrombus detection and removal from an implantable blood pump using a flexible electronic emitter and sensor.

### BACKGROUND

Mechanical Circulatory Support Devices ("MCSDs") are commonly used to assist the pumping action of a failing heart. Typically, an MCSD includes an implantable blood pump that is surgically implanted in a patient's body. More specifically, the MCSD includes a housing with an inlet, an outlet, and a rotor mounted therein. The inlet is connected to a chamber of the patient's heart, typically the left ventricle, whereas the outlet is connected to an artery, such as the aorta. Rotation of the rotor drives blood from the inlet towards the outlet and thus assists blood flow from the chamber of the heart into the artery. For example, the MCSD may be the HVAD^{®} Pump or the MVAD^{®} Pump, manufactured by HeartWare, Inc. in Miami Lakes, Fla., USA. The HVAD^{®} Pump is further discussed in U.S. Patent No. 8,512,013, the disclosure of which is hereby incorporated in its entirety. The MVAD^{®} Pump is further discussed in U.S. Patent Nos. 8,007,254 and 9,561,313.

Blood pumps used in MCSDs are desirably provided with contactless bearings so that, in operation, the rotor floats within the housing. With contactless bearings, there is no solid-to-solid contact between the rotor and the housing, and thus no mechanical wear during operation. One form of contactless bearing is a hydrodynamic bearing. In a hydrodynamic bearing, the liquid being pumped passes between a surface of the rotor and the surfaces of a hydrodynamic bearing which creates a clearance that is many times larger than the dimensions of blood cells. The surfaces are configured so that as the rotor turns, the fluid disposed between these surfaces exerts pressure on the surface of the rotor that holds the rotor away from the housing. However, in some cases the blood passing through the blood pump may contain particles of thrombus, a solid or semi-solid deposit generated within a patient's body. The thrombus can lodge on the surface of the hydrodynamic bearing and impede its operation. Unfortunately, known blood pumps fail to include a sensor or other mechanism to detect and/or remove the thrombus from the blood pump.

### SUMMARY

The invention is defined by independent claim 1.

The techniques of this disclosure generally relate to a method (not claimed) and system for thrombus detection and/or removal within a blood pump.

In one aspect, the system includes a housing having an inlet cannula, a rotor disposed within the housing, the rotor being in fluid communication with the inlet cannula, and a stator disposed within the housing, the stator configured to rotate the rotor when a current is applied to the stator. A flexible circuit assembly is also disposed within the housing, the flexible circuit assembly including at least one of the group consisting of a plurality of light emitters and a plurality of ultrasound emitters.

In another aspect, the disclosure provides a ceramic disk disposed between the stator and the rotor, the flexible circuit assembly being coupled to the ceramic disk.

In another aspect, the disclosure provides the flexible circuit assembly is coupled to the inner tube of the inlet cannula.

In another aspect, the disclosure provides the flexible circuit assembly is adhesively coupled to the inner tube of the inlet cannula.

In another aspect, the disclosure provides the flexible circuit assembly is a printed material fixedly coupled to the inner tube of the inlet cannula.

In another aspect, the disclosure provides the flexible circuit assembly includes the plurality of light emitters defining a light path and a plurality of light detectors in communication with the plurality of light emitters.

In another aspect, the disclosure provides a signal processor for processing at least one optical characteristic associated with the light path.

In another aspect, the disclosure provides the at least one optical characteristic is associated with a thrombus formation.

In another aspect, the disclosure provides the flexible circuit assembly includes a plurality of ultrasonic emitters for emitting an amount of acoustic energy.

In another aspect, the disclosure provides, the plurality of ultrasonic emitters are angled in a direction toward the rotor.

In another aspect, the disclosure provides the amount of acoustic energy is sufficient to dislodge a particle disposed within the housing.

In another aspect of this embodiment, the particle is thrombus.

In one aspect, the disclosure provides a method (not claimed) for detecting and removing a thrombus within a blood pump includes activating a plurality of light emitters disposed within a blood pump to shine a light onto at least one from the group consisting of a rotor and a portion of blood within the blood pump, the light defining a light path. The light path is detected and observed using a plurality of light detectors in communication with the plurality of light emitters. The light path is analyzed to determine a thrombus within the portion of blood.

In another aspect, the disclosure provides that the method further includes observing and recording the light path detected by the plurality of light detectors during a series of spaced apart time intervals.

In another aspect, the disclosure provides that the method further includes adjusting a wavelength of the plurality of light emitters.

In another aspect, the disclosure provides that the method further includes generating an alert corresponding to the detection of the thrombus.

In another aspect, the disclosure provides that the method further includes activating a plurality of ultrasound emitters disposed within the blood pump to shine an amount of acoustic energy onto the at least one from the group consisting of the rotor and the portion of blood within the blood pump when the thrombus is detected.

In another aspect, the disclosure provides that the method further includes activating the plurality of ultrasound emitters disposed within the blood pump to shine the amount of acoustic energy onto the at least one from the group consisting of rotor and the portion of blood within the blood pump during a series of time intervals to prevent a future thrombus formation.

In another aspect, the disclosure provides that the method further includes adjusting a frequency of the plurality of ultrasound emitters.

In another aspect, the disclosure provides that the method further includes adjusting an angle of the plurality of ultrasound emitters.

In one aspect, the present disclosure provides a system for thrombus detection and removal from a blood pump includes a housing including an inlet cannula, the inlet cannula having an inner tube. A rotor is disposed within the housing, the rotor being in fluid communication with the inlet cannula and the inner tube. A stator is disposed within the housing, the stator configured to rotate the rotor when a current is applied to the stator. A flexible circuit assembly includes at least one of the group consisting of a plurality of light emitters and a plurality of ultrasound emitters substantially surrounding the inner tube.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

A more complete understanding of embodiments described herein, and the attendant advantages and features thereof, will be more readily understood by reference to the following detailed description when considered in conjunction with the accompanying drawings wherein:
FIG. 1 is an exploded view of an exemplary blood pump constructed in accordance of the principles of the present application;
FIG. 2 is an exploded view of another exemplary blood pump constructed in accordance of the principles of the present application;
FIG. 3 is a downward looking cross-sectional view of an inlet cannula of the blood pump of FIG. 2; and
FIG. 4 is a downward looking cross-sectional view of the inlet cannula of the blood pump of FIG. 2 showing a particle of thrombus disposed therein.

### DETAILED DESCRIPTION

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before describing in detail exemplary embodiments, it is noted that the embodiments reside primarily in combinations of system components and processing steps related to thrombus detection and removal using a flexible circuit assembly. Accordingly, the system and method components have been represented where appropriate by conventional symbols in the drawings, showing only those specific details that are pertinent to understanding the embodiments of the present disclosure so as not to obscure the disclosure with details that will be readily apparent to those of ordinary skill in the art having the benefit of the description herein.

As used herein, relational terms, such as "first" and "second," "top" and "bottom," and the like, may be used solely to distinguish one entity or element from another entity or element without necessarily requiring or implying any physical or logical relationship or order between such entities or elements. The terminology used herein is for the purpose of describing particular embodiments only and is not intended to be limiting of the concepts described herein. As used herein, the singular forms "a", "an" and "the" are intended to include the plural forms as well, unless the context clearly indicates otherwise. It will be further understood that the terms "comprises," "comprising," "includes" and/or "including" when used herein, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this disclosure belongs. It will be further understood that terms used herein should be interpreted as having a meaning that is consistent with their meaning in the context of this specification and the relevant art and will not be interpreted in an idealized or overly formal sense unless expressly so defined herein.

In embodiments described herein, the joining term, "in communication with" and the like, may be used to indicate electrical or data communication, which may be accomplished by physical contact, induction, electromagnetic radiation, radio signaling, infrared signaling or optical signaling, for example. One having ordinary skill in the art will appreciate that multiple components may interoperate and modifications and variations are possible of achieving the electrical and data communication.

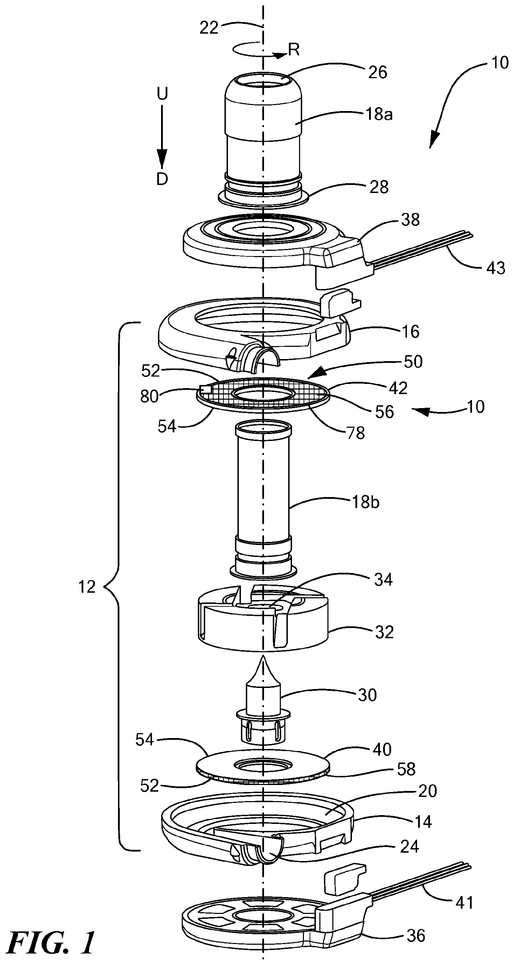

Referring now to the drawings in which like reference designators refer to like elements there is shown in FIG. 1 an exemplary blood pump constructed in accordance with the principles of the present application and designated generally "10." The blood pump 10, according to one embodiment of the disclosure, includes a static structure or housing 12 which houses the components of the blood pump 10. In one configuration, the housing 12 includes a lower housing or first portion 14, an upper housing or second portion 16, and an inlet portion or the inlet cannula 18 which includes an outer tube 18a and an inner tube 18b. The first portion 14 and the second portion 16 cooperatively define a volute-shaped chamber 20 having a major longitudinal axis 22 extending through the first portion and the inlet cannula 18. The chamber 20 defines a radius that increases progressively around the axis 22 to an outlet location on the periphery of the chamber 20. The first portion 14 and the second portion 16 define an outlet 24 in communication with the chamber 20. The first portion 14 and the second portion 16 also define isolated chambers (not shown) separated from the volute chamber 20 by magnetically permeable walls.

The inlet cannula 18 is generally cylindrical and extends from the first portion 14 generally along axis 22. The inlet cannula 18 has an upstream end or proximal end 26 remote from the second portion 16 and a downstream end or distal end 28 proximate the chamber 20. The parts of the housing 12 may be fixedly connected to one another so that the housing 12 as a whole defines a continuous enclosed flow path extending from the upstream end 26 to the outlet 24 at the downstream end 28. The upstream and downstream directions along the flow path are indicated by the arrows U and D respectively.

A post 30 is mounted to first portion 14 along axis 22. A generally disc-shaped ferromagnetic rotor 32 with a central hole 34 is mounted within the chamber 20 for rotation about the axis 22. The rotor 32 includes a permanent magnet and also includes flow channels for transferring blood from adjacent the center of the rotor 32 to the periphery of the rotor 32. In the assembled condition, the post 30 is received in the central hole of the rotor 32.

A first stator 36 having more than one coil may be disposed within the first portion 14 upstream from the rotor 32. The first stator 36 may be axially aligned with the rotor along axis 22 such that when a current is applied to the coils in the first stator 36, the electromagnetic forces generated by the first stator 36 rotate the rotor 32 and pump blood. A second stator 38 may be disposed within the second portion 16 downstream from the rotor 32. The second stator 38 may be configured to operate in conjunction with or independently of the first stator 36 to rotate the rotor 32.

An electrical connectors 41 and 43 (FIG.1) are provided on the first portion 14 and second portion 43, respectively for connecting the respective coils to a source of power such as a controller (not shown). The controller is arranged to apply power to the coils of the pump to create a rotating magnetic field which spins the rotor 32 around the axis 22 in a predetermined first direction of rotation, such as the direction R indicated by the arrow in FIG. 1, *i.e.,* counterclockwise as seen from the upstream end of the inlet cannula 18. In other configurations of the blood pump 10, the first direction may be clockwise. Rotation of the rotor 32 impels blood downstream along the flow path so that the blood moves in a downstream direction D along the flow path and exits through the outlet 24. During rotation, hydrodynamic and magnetic bearings (not shown) support the rotor 32 and maintain the rotor 32 out of contact with the surfaces of the elements of the first portion 14 and the second portion 16 during operation. The general arrangement of the components described above may be similar to the blood pump 10 used in the MCSD sold under the designation HVAD^{®} by HeartWare, Inc., assignee of the present application. The arrangement of components such as the magnets, electromagnetic coils, and hydrodynamic bearings used in such a pump and variants of the same general design are described in U.S. Patent Nos. 6,688,861; 7,575,423; 7,976,271; and 8,419,609.

A first non-ferromagnetic disk 40 may be disposed within the first portion 14 downstream from the rotor 32 between the first stator 36 and the rotor 32 and a second non-ferromagnetic disk 42 may be disposed upstream from the rotor 32 within the second portion 16 between the second stator 38 and the rotor 32. The first and second disks 40 and 42 may be composed of a ceramic material which is attached to the first portion 14 or the second portion 16 of the housing 12.

In one configuration, the blood pump 10 may include a system for thrombus detection and/or removal. The system includes flexible electronics, such as emitters and sensors, in the form a flexible circuit assembly 50 disposed within the housing 12. In one configuration, the flexible circuit assembly 50 may be coupled to the first disk 40 and/or the second disk 42 of the HVAD^{®}. In an alternative example, the circuit assembly 50 may be coupled to an inlet cannula, such as the inlet cannula of the MVAD^{®} Pump, as described in further detail below. As shown in FIG. 1, the first and second disks 40 and 42 may each include a first surface 52 facing away from the rotor 32 which may be sealed within the respective first portion 14 or the second portion 16 of the housing 12 out of the blood flow path. In other words, the circuit assembly 50 may be coupled to the first surface 52 so as to be out of contact with the blood. A second surface 54 is also shown opposite the first surface 52 facing toward the rotor 32.

In order to secure the circuit assembly 50 within the housing 12, the circuit assembly 50 may be configured as an adhesive substrate that is adhered to or stretched around the first surface 52 of the first disk 40 or the second disk 42. In another configuration, the circuit assembly 50 may be a printed material that is fixedly coupled to the first disk 40 and/or the second disk 42 using an etching process, printing process, or the like.

The circuit assembly 50 may include one or more light emitters 56 for shining a light onto the blood within the housing 12. In one configuration, the light is an infrared light. In other configurations, the light may be green, yellow, orange, red, or another wavelength or various wavelengths of the electromagnetic spectrum. The light may define a light path detectable by one or more light detectors 58, such photosensors, photodetectors, or the like, in communication with the light emitters 56. The light emitters 56 and the light detectors 58 may be referred to herein as the emitters 56 and the detectors 58. The wavelengths of the light path may be selectively adjusted or fixed. The emitters 56 and the detectors 58 may be coupled to the first disk 40 and/or the second disk 42 in communication with each other. The first disk 40 and the second disk 42 may be transparent and/or translucent to the light, thus allowing the light to shine through the first disk 40 and/or the second disk 42 onto the blood.

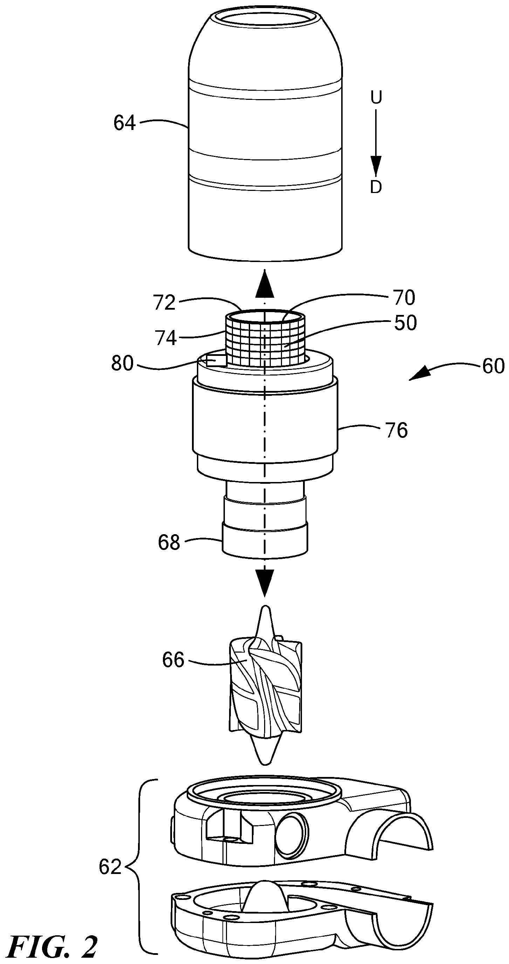

With reference to FIG. 2, in one configuration the circuit assembly 50 may be coupled within a blood pump 60, such as the MVAD^{®} Pump referenced above. For example, the blood pump 60 may include a housing 62 having an inlet cannula 64 and a rotor 66 proximate the inlet cannula 64 to impel the blood. The inlet cannula 64 may include an inner tube 68 formed from a non-magnetic material, such as a ceramic. The inner tube 68 includes an interior surface 70 defining a cylindrical bore 72 for receiving the rotor 66 therein. The inner tube 68 also includes a cylindrical outer surface 74 surround by a stator 76. The stator 76 includes pole pieces (not shown) which receive electrical current from a drive circuit (not shown) to spin the rotor 66, thus impelling the blood from an upstream direction U to a downstream direction D relative to the rotor 66 and the housing 62.

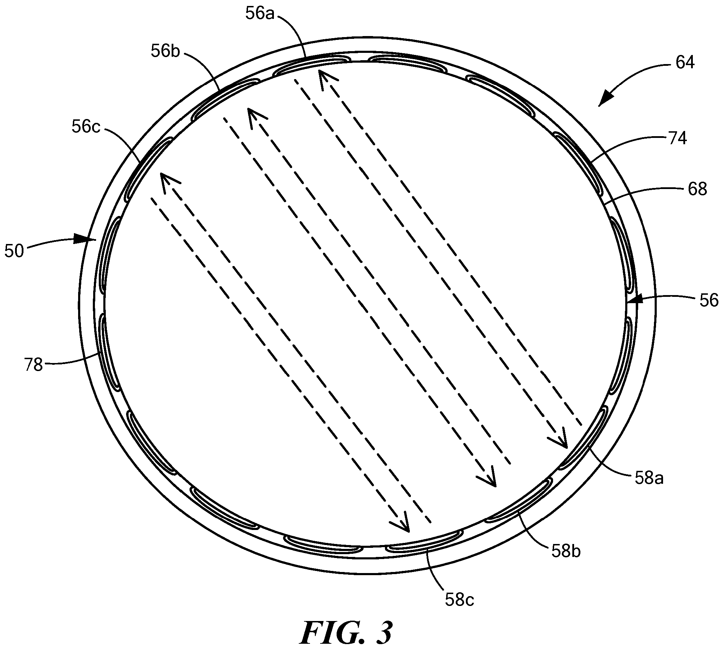

In one configuration, in both the blood pump 10 and the blood pump 60, the circuit assembly 50 may be coupled to the outer surface 74 of the inner tube 68 using the methods described above with respect to the first disk 40 and the second disk 42 of the blood pump 10. For example, as shown in FIG. 3 depicting a downward looking cross-sectional view of the inlet cannula 64, the circuit assembly 50 may be coupled to the outer surface 74 of the inner tube 68 out of contact with the blood flow path. In the alternative, the circuit assembly 50 may be embedded within the inner tube 68. In such configurations, the emitters 56a-56c may shine light detectable by the detectors 58a-58c arranged around the inner tube 68. The inner tube 68 may be transparent and/or translucent to the light, thus allowing the light to shine through onto the blood. The emitters 56 and the detectors 58 may be selectively activated, depending upon the thrombus detection strategy. In addition, the emitters 56 and the detectors 58 may be arranged such that light is transmitted across the inner tube 68, reflected from the rotor 66, and/or reflected from a tissue that may obscure the light shining across the inner tube 68.

In one configuration, the system may include a signal processor (not shown) configured to process an optical characteristic associated with the light path observed by the detectors 58. In the alternative, the signal processor may be configured to process a thermal characteristic associated with the light, such as when the light is an infrared light. The signal processor may be an external processor (not shown) in communication with the circuit assembly 50 through the coils. In the alternative, the signal processor may utilize wireless transmission or may be disposed within the housing 12, 62.

The optical characteristic and/or the thermal characteristic associated with the light path may be associated with abnormal blood flow through the pump 10, 60 or an abnormal blood or tissue characteristic that is indicative of thrombus. For example, because thrombus typically begins to form in a radial gap (not shown), which may be for example between the rotor 32 and the housing 12 in the blood pump 10, when thrombus is present, the opacity and/or light transmission characteristics of the blood or tissue in the radial gap may change. The change in opacity and/or light transmission can be detected by the detectors 58 and processed by the signal processor to determine the presence of the thrombus or the onset of the thrombus formation. An alert, such as an audio or visual alert, may be sent to the controller (not shown) upon thrombus detection.

The reflected light path, including the change in opacity and/or light transmission, may be observed and recorded during a series of spaced apart time intervals, such as hourly, daily or the like. The recorded information may be used to adjust a wavelength of the emitters 56 to detect the thrombus. The signal processor and recorded information may also be used to determine an estimate of the blood's hematocrit and oxygen saturation or may provide a measurement of the rotor speed.

With reference to FIGS. 1 and 3, in addition to or in lieu of the emitters 56 and the detectors 58, the circuit assembly 50 may include one or more ultrasonic emitters 78 for emitting acoustic energy to remove or prevent the thrombus. For example, the amount of acoustic energy emitted may be tuned and adjusted to frequencies optimal for dislodging existing thrombus or cleaning the surface of the rotor 32, 66 for preventing future thrombus. As described above with respect to the emitters 56, the ultrasonic emitters 78 may be coupled to the first disk 40 and/or the second disk 42 of the blood pump 10 and/or the inner tube 68 of the inlet cannula 64 of the blood pump 60 by adhesion, etching, printing, or the like. The ultrasonic emitters 78 may be angled in a direction toward the rotor 32, 66 and a portion of blood within the blood pump 10, 60 such as a portion of blood within the radial gap (not shown) containing the thrombus. The angle may be adjusted in accordance with the flow path. In one configuration, the circuit assembly 50 may include the emitters 56, the detectors 58, and the ultrasonic emitters 78 spanning an entire length of the inner tube 68 in order to detect one or more particles of thrombus that may develop within the rotor 66 when the rotor 66 is disposed within the inner tube 68. The emitters 56, the detectors 58, and the ultrasonic emitters 78 may be disposed in various configurations around the inner tube 68.

The ultrasonic emitters 78 may be activated upon thrombus detection or during a series of time intervals, such as hourly, daily, or the like, to prevent the future thrombus formation. Such periodic activation may assist with keeping the rotor 32, 66 clean to prevent accumulation of deposits that could lead to the thrombus.

In one configuration, the signal processor (not shown) may be configured to receive the information associated with the reflected light path from the emitters 56 and the detectors 58 and thereafter automatically activate the ultrasonic emitters 78 when the thrombus is detected. For example, the signal processor may be preprogrammed to activate the ultrasonic emitters 78 when the changes in the opacity and/or light transmission depart from a threshold. In another configuration, the signal processor may be programmed to activate the ultrasonic emitters 78 during a time interval, such as hourly, daily, or as otherwise determined in accordance with the individual patient. In another example, the signal processor may be preprogrammed to activate the ultrasonic emitters 78 when the detectors 58 sense that a temperature within the blood pump 10 departs from a temperature threshold.

Referring again to FIGS. 1 and 2, the circuit assembly 50 may be in communication with a source of power 80, such as a battery or electricity. For example, the emitters 56 and/or the ultrasonic emitters 78 may be wired to a battery disposed within the housing 12, 62. The battery may be rechargeable by a transcutaneous energy transfer (TET) system or another charging method. In another configuration, the source of power 80 may be electricity provided through one or more of the coils of the electrical connector 41 (FIG. 1) connected to the controller (not shown). In the alternative, the source of power 80 may be a driveline separate from the electrical connector 41 which connects to the controller or another external power source.

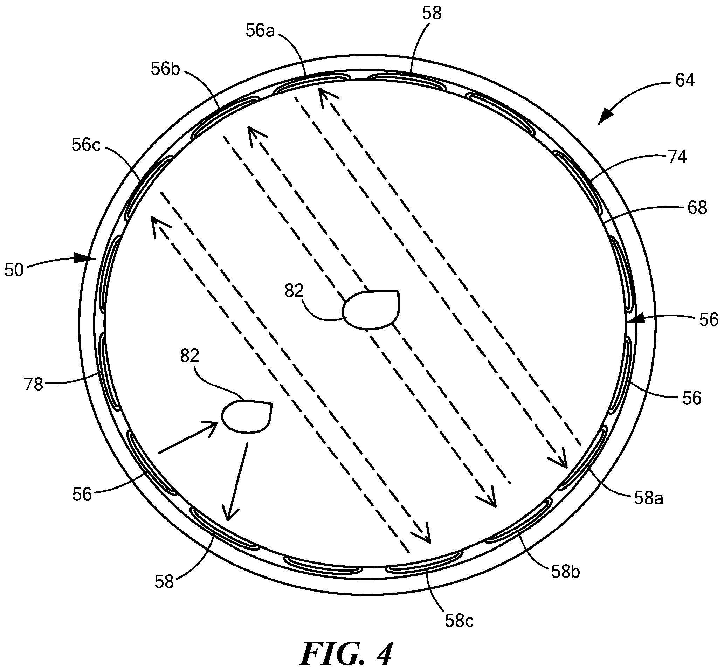

With reference to FIG. 4, one or more particles of thrombus 82 may be disposed within the inner tube 68 of the inlet cannula 64. Upon activation, the emitters 56 may shine the light onto the particle of thrombus 82. The light path may be reflected from the particle of thrombus 82 and detected by the detector 58. In one configuration, the signal processor (not shown) may receive the information associated with the reflected light path from detector 58 and provide an indication that the light path is abnormal due to the presence of the particle of thrombus 82. The ultrasonic emitter 78 may then be activated to remove the particle of thrombus 82.

## Claims

1. A system for thrombus detection and/or removal from a blood pump (10) comprising:
a housing (12) including an inlet cannula (18), the inlet cannula (18) having an inner tube (18b);
a rotor (32) disposed within the housing (12), the rotor (32) being in fluid communication with the inlet cannula (18);
a stator (36) disposed within the housing, the stator (36) configured to rotate the rotor (32) when a current is applied to the stator (36); and **characterised by**
a flexible circuit assembly (50) disposed within the housing (12), the flexible circuit assembly including at least one of the group consisting of a plurality of light emitters (56) and a plurality of ultrasound emitters (78).

2. The system of Claim 1, further comprising a ceramic disk (40) disposed between the stator (36) and the rotor (32), the flexible circuit assembly (50) being coupled to the ceramic disk (40).

3. The system of Claims 1 or 2, wherein the flexible circuit assembly (50) is coupled to the inner tube (18b) of the inlet cannula (18).

4. The system of Claim 3, wherein the flexible circuit assembly (50) is adhesively coupled to the inner tube (18b) of the inlet cannula (18).

5. The system of Claim 3, wherein the flexible circuit assembly (50) is a printed material fixedly coupled to the inner tube (18b) of the inlet cannula (18).

6. The system of any one of Claims 1-5, wherein the flexible circuit assembly (50) includes the plurality of light emitters (56) defining a light path and a plurality of light detectors (58) in communication with the plurality of light emitters (56).

7. The system of Claim 6, further comprising a signal processor for processing at least one optical characteristic associated with the light path.

8. The system of Claim 7, wherein the at least one optical characteristic is associated with a thrombus formation.

9. The system of any one of Claims 1-8, wherein the flexible circuit assembly (50) includes a plurality of ultrasonic emitters (78) for emitting an amount of acoustic energy.

10. The system of Claim 9, wherein the plurality of ultrasonic emitters (78) are angled in a direction toward the rotor (32).

11. The system of Claim 10, wherein the amount of acoustic energy is sufficient to dislodge a particle disposed within the housing (12).

12. The system of Claim 11, wherein the particle is thrombus.

## Patentansprüche

1. System für eine Detektierung und/oder eine Entfernung von einem Thrombus aus einer Blutpumpe (10), umfassend:
ein Gehäuse (12), das eine Einlasskanüle (18) einschließt, wobei die Einlasskanüle (18) ein Innenrohr (18b) aufweist;
einen Rotor (32), der innerhalb des Gehäuses (12) angeordnet ist, wobei der Rotor (32) in Fluidverbindung mit der Einlasskanüle (18) steht;
einen Stator (36), der innerhalb des Gehäuses angeordnet ist, wobei der Stator (36) konfiguriert ist, um den Rotor (32) zu rotieren, wenn dem Stator (36) Strom zugeführt wird; und **gekennzeichnet durch**
eine flexible Schaltungsanordnung (50), die innerhalb des Gehäuses (12) angeordnet ist, wobei die flexible Schaltungsanordnung mindestens eines der Gruppe einschließt, bestehend aus einer Vielzahl von Lichtemittern (56) und einer Vielzahl von Ultraschallemittern (78).

2. System nach Anspruch 1, ferner umfassend eine Keramikscheibe (40), die zwischen dem Stator (36) und dem Rotor (32) angeordnet ist, wobei die flexible Schaltungsanordnung (50) mit der Keramikscheibe (40) gekoppelt ist.

3. System nach Anspruch 1 oder 2, wobei die flexible Schaltungsanordnung (50) mit dem Innenrohr (18b) der Einlasskanüle (18) gekoppelt ist.

4. System nach Anspruch 3, wobei die flexible Schaltungsanordnung (50) mit dem Innenrohr (18b) der Einlasskanüle (18) haftend gekoppelt ist.

5. System nach Anspruch 3, wobei die flexible Schaltungsanordnung (50) ein gedrucktes Material ist, das mit dem Innenrohr (18b) der Einlasskanüle (18) fest gekoppelt ist.

6. System nach einem der Ansprüche 1 bis 5, wobei die flexible Schaltungsanordnung (50) die Vielzahl von Lichtemittern (56) einschließt, die einen Lichtpfad und eine Vielzahl von Lichtdetektoren (58) in Kommunikation mit der Vielzahl von Lichtemittern (56) definiert.

7. System nach Anspruch 6, ferner umfassend einen Signalprozessor zum Verarbeiten mindestens einer optischen Eigenschaft, die dem Lichtpfad zugeordnet ist.

8. System nach Anspruch 7, wobei die mindestens eine optische Eigenschaft einer Thrombusbildung zugeordnet ist.

9. System nach einem der Ansprüche 1 bis 8, wobei die flexible Schaltungsanordnung (50) eine Vielzahl von Ultraschallemittern (78) zum Emittieren einer Menge an Schallenergie einschließt.

10. System nach Anspruch 9, wobei die Vielzahl von Ultraschallemittern (78) in einer Richtung zu dem Rotor (32) hin abgewinkelt sind.

11. System nach Anspruch 10, wobei die Menge an Schallenergie ausreicht, um ein Teilchen zu lösen, das innerhalb des Gehäuses (12) angeordnet ist.

12. System nach Anspruch 11, wobei das Teilchen der Thrombus ist.

## Revendications

1. Système pour la détection et/ou le retrait de thrombus d'une pompe à sang (10) comprenant :
un boîtier (12) comportant une canule d'entrée (18), la canule d'entrée (18) ayant un tube interne (18b) ;
un rotor (32) disposé à l'intérieur du boîtier (12), le rotor (32) étant en communication fluidique avec la canule d'entrée (18) ;
un stator (36) disposé à l'intérieur du boîtier, le stator (36) étant configuré pour faire tourner le rotor (32) lorsqu'un courant est appliqué au stator (36) ; et **caractérisé par**
un ensemble de circuit flexible (50) disposé à l'intérieur du boîtier (12), l'ensemble de circuit flexible comportant au moins l'un du groupe constitué d'une pluralité d'émetteurs de lumière (56) et d'une pluralité d'émetteurs d'ultrasons (78).

2. Système selon la revendication 1, comprenant en outre un disque céramique (40) disposé entre le stator (36) et le rotor (32), l'ensemble de circuit flexible (50) étant accouplé au disque céramique (40).

3. Système selon les revendications 1 ou 2, dans lequel l'ensemble de circuit flexible (50) est accouplé au tube interne (18b) de la canule d'entrée (18).

4. Système selon la revendication 3, dans lequel l'ensemble de circuit flexible (50) est accouplé de manière adhésive au tube interne (18b) de la canule d'entrée (18).

5. Système selon la revendication 3, dans lequel l'ensemble de circuit flexible (50) est un matériau imprimé accouplé à demeure au tube interne (18b) de la canule d'entrée (18).

6. Système selon l'une quelconque des revendications 1 à 5, dans lequel l'ensemble de circuit flexible (50) comporte la pluralité d'émetteurs de lumière (56) définissant un trajet de lumière et une pluralité de détecteurs de lumière (58) en communication avec la pluralité d'émetteurs de lumière (56).

7. Système selon la revendication 6, comprenant en outre un processeur de signal permettant de traiter au moins une caractéristique optique associée au trajet de lumière.

8. Système selon la revendication 7, dans lequel l'au moins une caractéristique optique est associée à une formation de thrombus.

9. Système selon l'une quelconque des revendications 1 à 8, dans lequel l'ensemble de circuit flexible (50) comporte une pluralité d'émetteurs ultrasonores (78) permettant d'émettre une quantité d'énergie acoustique.

10. Système selon la revendication 9, dans lequel la pluralité d'émetteurs ultrasonores (78) sont inclinés dans une direction vers le rotor (32).

11. Système selon la revendication 10, dans lequel la quantité d'énergie acoustique est suffisante pour déloger une particule disposée à l'intérieur du boîtier (12).

12. Système selon la revendication 11, dans lequel la particule est un thrombus.
